Europäisches Patentamt

European Patent Office

Office européen des brevets

(11) Publication number: **0 261 729**
**A1**

## EUROPEAN PATENT APPLICATION

(21) Application number: 87201773.6

(22) Date of filing: 16.09.87

(51) Int. Cl.4: **B01J 45/00** , C07D 235/18 , C22B 15/00

(30) Priority: 22.09.86 NL 8602391

(43) Date of publication of application:
30.03.88 Bulletin 88/13

(84) Designated Contracting States:
AT BE CH DE ES FR GB GR IT LI LU NL SE

(71) Applicant: **Rijksuniversiteit te Leiden**
**Stationsweg 46**
**NL-2312 AV Leiden(NL)**

(72) Inventor: **Reedijk, Jan**
**Antonie Duycklaan 4**
**NL-2334 CD Leiden(NL)**

(74) Representative: **van der Beek, George Frans**
**et al**
**Nederlandsch Octrooibureau Johan de**
**Wittlaan 15 P.O. Box 29720**
**NL-2502 LS 's-Gravenhage(NL)**

(54) **Method for preparing a ion exchanger, method for the removal and separation of copper from a solution, the separation of copper from a solution of metal ions.**

(57) The invention relates to a method for preparing an ion exchanger,.to be used for the removal and separation of copper from a solution, especially a solution containing metal ions. The ion exchanger of the invention comtains a benzimidazole group as the active group. Such an ion exchanger can be prepared by reacting an ion exchanger which has a suitable reactive group with o-phenylenediamine.

fig. 6.

$$D = \frac{mg\ METAL/g\ DRY\ RESIN}{mg\ METAL/mL\ SOLUTION}$$

LOG D

o CuCl$_2$
□ Cu(NO$_3$)$_2$

RESIN ES 466B

pH

EP 0 261 729 A1

## Method for preparing an ion exchanger, method for the removal and separation of copper from a solution, the separation of copper from a solution of metal ions.

The invention relates to an ion exchanger, to a method for the preparation of an ion exchanger to a method for the removal and separation of copper from a solution, and also to a method for the separation of copper from other metals in a solution.

The ion exchangers most used exhibit no selectivity for binding a specific metal. So-called chelating ion exchangers do this to a certain extent. They bind, as a rule, heavy metals selectively with respect to, for example, alkali and alkaline earth metals, but the selectivity of the heavy metals with respect to each other is very limited. These known chelating ion exchangers have iminodiacetic acid as active group.

An ion exchangr has now been found which is characterized in that it contains a benzimidazole group as active group. Such an ion exchanger can be prepared by reacting an ion exchanger which has a suitable reactive group with o-phenylenediamine. Such an ion exchanger exhibits a high specific activity for copper and is capable of removing copper selectively from a solution which also contains one or more other metal ions in addition to copper.

A suitable reactive group is the iminodiacetic acid group. In that case, 220-260°C is a suitable reaction temperature. The reaction time must be sufficiently long to ensure a good reaction.

On theoretical grounds it was not to be expected that an ion exchanger containing benzimidazole groups would exhibit a preference for a divalent copper over other divalent (transition) metals. The reason for this is that many stable coordination compounds of imidazole derivatives (including, of course, benzimidazole) with divalent transition metal ions are known from the scientific literature.

Some examples of transition metal ions ($Fe^{3+}$, $Fe^{2+}$, $Mn^{2+}$, $Co^{2+}$, $Ni^{2+}$, $Cu^{2+}$, $Zn^{2+}$, $Ag^{2+}$, $Cd^{2+}$, $Pd^{2+}$, and $Pt^{2+}$) with (benz)imidazole ligands are given in the publications below:

-J. Reedijk, J.Inorg.Nucl.Chem. 1973, 35, 239;
-R.J. Sundberg and R.B. Martin, Chem. Rev. 1974, 74, 472;
-J. Reedijk and J.K. de Ridder, Inorg.Nucl.Chem.Letters 1976, 12, 585;
-H.M.J. Hendriks, W.O. ten Bokkel Huinink and J. Reedijk, Rec.Trav.Chim. Pays-Bas 1979, 98, 499;
-P.Gomez-Romero, G.C.DeFotis and G.B. Jameson, J.Am.Chem. Soc. 1986, 108, 851;
-W. Clegg, J.C.Lockhart and F.H. Musa, J.C.S. Dalton Trans. 1986, 47.

### Example of Preparation

20 g of ES466 resin manufactured by Duolite International and containing nitriloacetate groups are introduced into one and a half litres of 0.5 M hydrochloric acid and kept there for one night. After being decantered, the resin is washed with water and placed under water for one night. Then the resin is washed successively with methanol and ether and finally dried under reduced pressure at 60°C.

A solution of 13 g of 1,2-diaminobenzene in 200 ml of ethylene glycol is added to 10 g of the treated resin. This mixture is stirred for 65 hours and heated in a manner such that the solution boils under reflux (boiling point of ethylene glycol 197°C). The hot solution is then decantered from the resin. The resin is then successively washed with dioxane, water, methanol and ether, whereafter the resin is dried under reduced pressure at 60°C. Yield: approximately 11 g of modified resin.

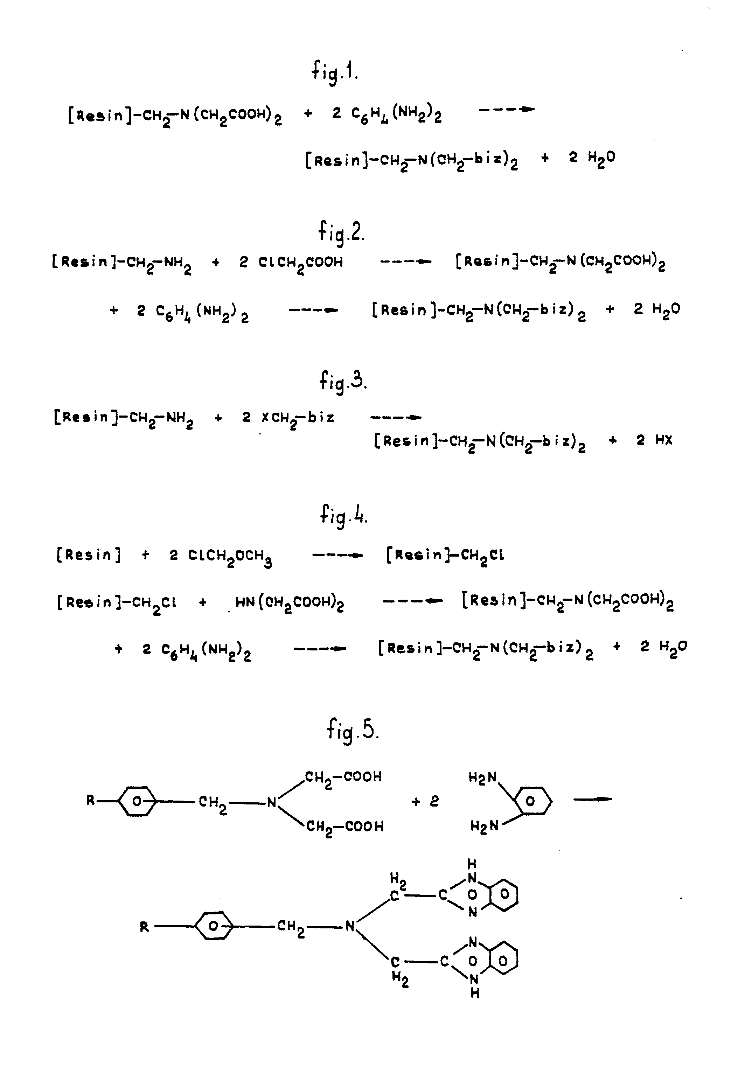

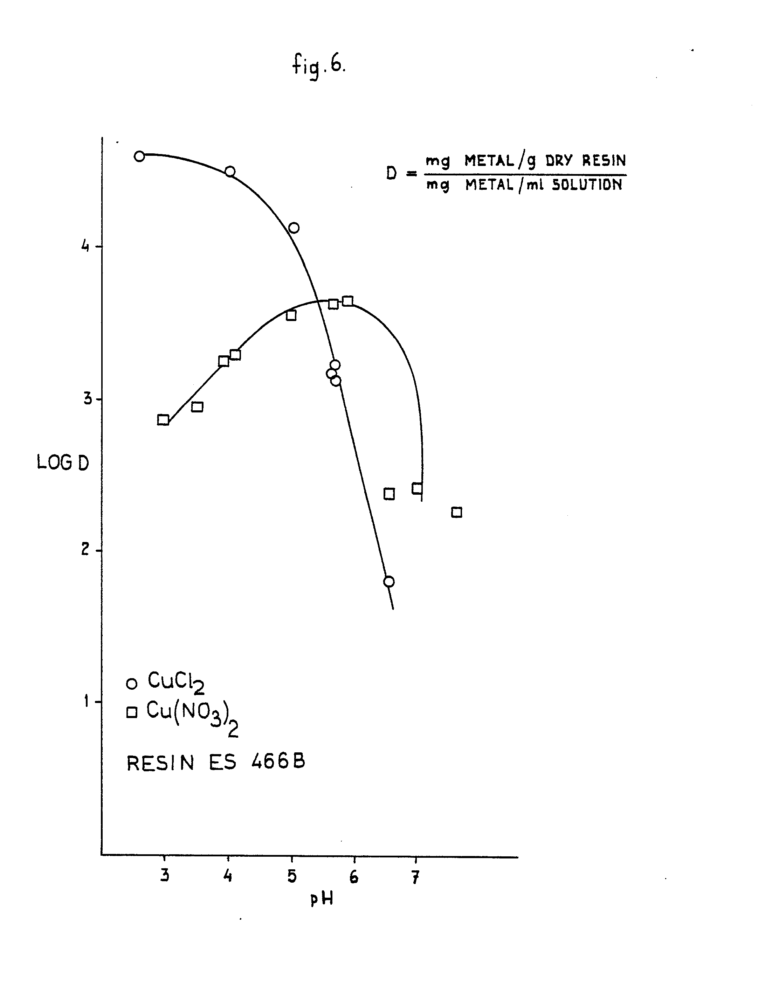

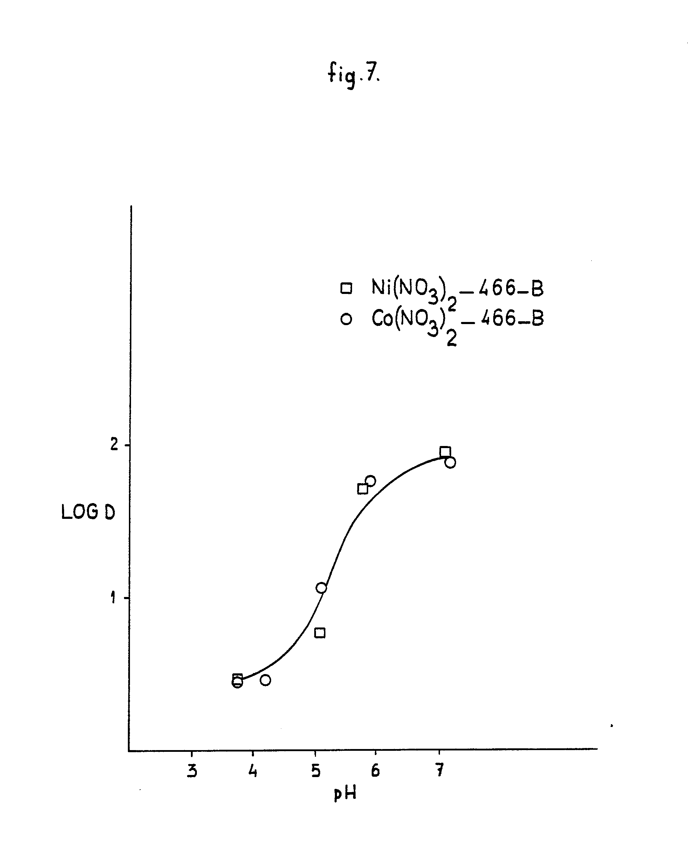

The reaction may be represented by Figure 1 on the formula page in which [resin] represents the polymer matrix of the ion exchanger, biz represents the benzimidazole group and X represents a halogen atom.

The reaction of the ion exchanger with a non-chelating active group may be represented by formula 2 or 3 in which the symbols have the above meanings.

In case a polystyrene is the starting point for the preparation, the reaction equation may be represented by Figure 4.

Application Example 1

100 g of a chelating ion exchanger containing iminodiacetic acid as active group is heated in an inert atmosphere for 60 hours at 240°C together with a two-or three-fold excess of o-phenylenediamine in ethylene glycol (any other suitable solvents may, of course, also be used, such as, for example, propylene glycol). After this treatment, the ion exchanger is filtered off and washed with alcohol (water may also be used). The reaction which occurs is represented in Figure 5 in which R represents the ion exchanger matrix.

The selectivity of the ion exchanger concerned is apparent from the comparison of the distribution coefficient of various metals or from comparison of the percentage of different metals present in solution which are bound by the ion exchanger.

The distribution coefficient is defined as:

$$D = \frac{\text{quantity of metal bound per gram of exchanger}}{\text{quantity of metal dissolved per ml of solution}}$$

Application Example 2

A number of ion exchangers containing benzimidazole as active group were investigated for distribution coefficient in separate solutions of copper (II), cobalt and nickel as shown in Figures 6 and 7. The high selectivity for copper is apparent, particularly in the acid range from pH 3 to 5. The values pertaining to Figures 6 and 7 are also shown in table A.

Table A

Metal absorption capacity of ES 466B compared with other elements

| Mixture of metal ions $(Cu^{2+}-M^{n+})$ | pH | % Cu | % M |
|---|---|---|---|
| $(Cu^{2+}-Fe^{3+})$[1] | 1.0 | 0.04 | 0.04 |
| | 1.5 | 0.19 | 0.04 |
| | 2.6 | 0.78 | 0.04 |
| | 3.0 | 1.20 | 0.04 |

| | | $(Cu^{2+})$ | $(M^{2+})$ |
|---|---|---|---|
| | | mg/g of wet resin | |
| $(Cu^{2+}-Co^{2+})$[2] | 5.5 | 27.3 | 0.0 |
| $(Cu^{2+}-Ni^{2+})$ | 5.5 | 30.4 | 0.0 |
| $(Cu^{2+}-Zn^{2+})$ | 5.5 | 26.5 | 0.0 |
| $(Cu^{2+}-Mg^{2+})$ | 5.5 | 26.7 | 0.0 |
| $(Cu^{2+}-Ca^{2+})$ | 4.0 | 26.1 | 0.0 |

[1]  These metal constants represent the percentage by weight as found in the analysis of polymer pellets containing metal.

[2]  These values are based on analysis of the solution containing the metal.

Table B lists some important properties of ES 466B

Table B

| | |
|---|---|
| Particle or pellet size: | 0,4 - 0,9 mm |
| Total copper (II) capacity: | 33-34 g/kg |
| Water content: | 50 - 55% |
| Volume change: | |
| Resin-Cu to resin (free): | negligible |
| Resin (base) as percentage of resin (acid): | approx. 25-30% |
| Elution of metal ions: | with ammonium hydroxide or with sulphuric acid |

Table C gives the elementary analysis of chelating resins and some copper (II) complexes of ES 466B.

Table C

| Compound | Colour | % C | % H | % N | % O |
|---|---|---|---|---|---|
| | | | (detected) | | |
| ES 466B (from ES-466) | Light yellow | 75.10 | 7.18 | 8.83 | 9.26 |
| Chelex-100B (from Chelex-100) | yellow | 77.65 | 6.39 | 9.94 | - |
| UR-50B (from UR-50) | dark green | 68.38 | 5.56 | 5.18 | - |
| $CuCl_2$-BS 466B* (capacity determined at pH = 5.5) | yellowish green | 71.59 | 6.37 | 7.99 | - |
| $CuCl_2$-ES 466B (dist. coeff. determined at pH = 7.0) | yellowish green | 73.61 | 6.22 | 8.88 | - |
| $CuNO_3$-ES 466B (dist. coeff. determined at pH = 7.0) | yellowish green | 73.23 | 6.79 | 8.84 | - |

\* It was found that the percentage of chlorine in the copper chloride complexes is less than 0.5%.

## Claims

1. Ion exchanger characterized in that it contains a benzimidazole group as active group.

2. Method for preparing an ion exchanger, characterized in that an ion exchanger is prepared which contains benzimidazole as active group.

3. Method according to Claim 2, characterized in that an existing ion exchanger which contains iminodiacetic acid as active group is reacted with o-phenylenediamine.

4. Method according to Claim 2, characterized in that an ion exchanger containing a nitrilodiacetate group as active group is reacted with o-phenylenediamine.

5. Method according to Claim 2, characterized in that the starting point is a polystyrene which contains a -CH₂NH₂-group.

6. Method according to Claims 1-5, characterized in that an ion exchanger is prepared which contains benzimidazole groups in a concentration of 0.2 mmol equivalent per gram.

7. Method according to Claims 1-6, characterized in that an ion exchanger is prepared having an exchange capacity of at least 20 g of copper (II) per kg.

8. Method for removing and separating copper with an ion exchanger, characterized in that an ion exchanger according to Claims 1-7 are used for the process.

9. Method according to Claim 8, characterized in that copper is removed from waste water or a current of circulating rinsing water.

10. Method according to Claim 8, characterized in that said method is applied to a liquid in a process current.

11. Method for removing or separating copper, characterized in that an ion exchanger to be prepared as described in Claims 1-7 is used in a hydrometallurgical extraction of copper from copper-containing ore.

## fig.1.

[Resin]—CH$_2$—N(CH$_2$COOH)$_2$ + 2 C$_6$H$_4$(NH$_2$)$_2$ ----→

[Resin]—CH$_2$—N(CH$_2$—biz)$_2$ + 2 H$_2$O

## fig.2.

[Resin]—CH$_2$—NH$_2$ + 2 ClCH$_2$COOH ----→ [Resin]—CH$_2$—N(CH$_2$COOH)$_2$

+ 2 C$_6$H$_4$(NH$_2$)$_2$ ----→ [Resin]—CH$_2$—N(CH$_2$—biz)$_2$ + 2 H$_2$O

## fig.3.

[Resin]—CH$_2$—NH$_2$ + 2 XCH$_2$—biz ----→

[Resin]—CH$_2$—N(CH$_2$—biz)$_2$ + 2 HX

## fig.4.

[Resin] + 2 ClCH$_2$OCH$_3$ ----→ [Resin]—CH$_2$Cl

[Resin]—CH$_2$Cl + HN(CH$_2$COOH)$_2$ ----→ [Resin]—CH$_2$—N(CH$_2$COOH)$_2$

+ 2 C$_6$H$_4$(NH$_2$)$_2$ ----→ [Resin]—CH$_2$—N(CH$_2$—biz)$_2$ + 2 H$_2$O

## fig.5.

fig.6.

$$D = \frac{mg\ METAL/g\ DRY\ RESIN}{mg\ METAL/ml\ SOLUTION}$$

LOG D

o CuCl$_2$
□ Cu(NO$_3$)$_2$

RESIN ES 466B

pH

fig.7.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | EP-A-0 126 231 (ASAHI)<br>* Page 2, lines 1-11; page 14, lines 12-14; page 75, claim 44; page 63, claim 12; page 65, claim 19 *<br>--- | 1,2,8,9 | B 01 J 45/00<br>C 07 D 235/18<br>C 22 B 15/00 |
| A | EP-A-0 036 584 (RIEDEL-DE HAEN)<br>* Page 4, line 4; pages 13,14 *<br>--- | 3 | |
| A | CHEMICAL ABSTRACTS, vol. 87, 1977, page 64, abstract no. 69573c, Columbus, Ohio, US; V.N. KOLOT et al.: "New chelate-forming fiber", & KHIM. VOLOKNA 1977, (3), 67-8<br>* Abstract *<br>--- | 1 | |
| A | CHEMICAL ABSTRACTS, vol. 75, 1971, page 446, abstract no. 63685w, Columbus, Ohio, US; V.I. ISAGULYANTS et al.: "Synthesis of 2-substituted benzimidazoles with the use of KU-2 cation exchange resin", & ZH. PRIKL. KHIM. 1971, 44(5), 1195-7<br>* Abstract *<br>----- | 3 | TECHNICAL FIELDS SEARCHED (Int. Cl.4)<br><br>B 01 J<br>C 07 D |

The present search report has been drawn up for all claims

| Place of search | Date of completion of the search | Examiner |
|---|---|---|
| THE HAGUE | 16-12-1987 | WENDLING J.P. |